# EUROPEAN PATENT SPECIFICATION

(11) **EP 4 194 038 B1**
(45) Date of publication and mention of the grant of the patent: **01.10.2025**
(21) Application number: 22211911.7
(22) Date of filing: 07.12.2022
(51) Int. Cl.: A61M 11/04

(54) **VAPORIZATION STATE RECOGNITION METHOD, APPARATUS, ELECTRONIC DEVICE AND STORAGE MEDIUM**
VERFAHREN ZUR ERKENNUNG EINES VERDAMPFUNGSZUSTANDS, VORRICHTUNG, ELEKTRONISCHES GERÄT UND SPEICHERMEDIUM
PROCÉDÉ DE RECONNAISSANCE DE L'ÉTAT DE VAPORISATION, APPAREIL, DISPOSITIF ÉLECTRONIQUE ET SUPPORT DE STOCKAGE

(30) Priority: 10.12.2021 CN 202111506032
(43) Date of publication of application: 14.06.2023
(73) Proprietor: Shenzhen Moore Vaporization Health & Medical Technology Co., Ltd., Shenzhen, Guangdong (CN)
(72) Inventor: YANG, Sheng, Shenzhen (CN)
(74) Representative: Herrero & Asociados, S.L.

(56) References cited:
- WO-A1-2016/062168
- WO-A1-2019/192226
- WO-A1-2021/238627

## Description

### TECHNICAL FIELD

This application relates to the field of electronic vaporization technologies, and in particular, to a vaporization state recognition method, an apparatus, an electronic device, and a storage medium.

### BACKGROUND

With the development of medical technologies, a vaporization inhalation therapy appears, and is usually used in the treatment of respiratory diseases. By means of an atomizing sheet of an electronic vaporization device, medicinal liquid is vaporized into tiny particles, and the vaporized medicine is inhaled and deposited in the respiratory tract and the lung of a patient through breathing, which can directly reach a lesion, accelerate the action of the medicine, so as to achieve a painless, rapid, and effective treatment.

Dry heating is a common problem during using the electronic vaporization device. Long-term dry heating damages the atomizing sheet, resulting in a decrease in performance, a reduction in a vaporization amount, and even a loss of a vaporization function for the atomizing sheet.

International Publication Number WO2019/192226A1 relates to an aerosol generating device and control method thereof. After one suction is completed and before the next suction is started, dry burning detection is performed. Before dry burning detection is performed, the temperature/resistance value of a heating component (300) is detected, and dry burning detection is performed only when the temperature/resistance value of the heating component (300) is less than a set temperature/resistance value. By arranging dry burning detection during a suction interval of a user, dry burning detection is not performed during user suction, thereby solving the delay effect. Moreover, dry burning detection is performed before the next suction of the user, thereby having the effect of preheating the next suction. The user experience is good.

### SUMMARY OF THE DISCLOSURE

A vaporization state recognition method, an apparatus, an electronic device and a storage medium provided by this application, resolve a technical problem about how to detect dry heating of an electronic vaporization device in the prior art.

To resolve the above technical problem, a first technical solution provided by this application is to provide a vaporization state recognition method as set out in the appended claim 1.

To resolve the above technical problem, a second technical solution provided by this application is to provide a vaporization state recognition apparatus as set out in the appended claim 7.

To resolve the above technical problem, a third technical solution provided by this application is to provide an electronic device, including: a memory and a processor, where the memory stores program instructions, and the processor reads the program instructions from the memory to execute the vaporization state recognition method according to any one of the above.

To resolve the above technical problem, a fourth technical solution provided by this application is to provide a computer-readable storage medium as set out in the appended claim 12.

Beneficial effects of this application: different from the prior art, this application discloses a vaporization state recognition method, an apparatus, an electronic device and a storage medium. The vaporization state recognition method including: obtaining a heating rate of an atomizing sheet of a vaporizer; and determining a vaporization state of the vaporizer according to the heating rate. Therefore, whether the vaporizer is dry heating may be recognized in time and the dry heating of the vaporizer may be dealt with in time. In addition, during recognizing the vaporization state of the vaporizer, there is no need to use a sensor such as a probe to contact medicinal liquid, so that a detection is more accurate.

### BRIEF DESCRIPTION OF THE DRAWINGS

To describe the technical solutions in the embodiments of this application more clearly, the following briefly introduces the accompanying drawings required for describing the embodiments. Apparently, the accompanying drawings in the following description show merely some embodiments of this application, and a person of ordinary skill in the art may still derive other accompanying drawings from these accompanying drawings without creative efforts.
FIG. 1 is a schematic flowchart of a first embodiment of a vaporization state recognition method according to this application.
FIG. 2 is a schematic flowchart of step S2 in the vaporization state recognition method according to FIG. 1.
FIG. 3 is a temperature rise curve diagram of an atomizing sheet in normal vaporization according to this application.
FIG. 4 is a schematic flowchart of step S2 of a second embodiment of a vaporization state recognition method according to this application.
FIG. 5 is a temperature rise curve comparison diagram of an atomizing sheet in different vaporization states according to this application.
FIG. 6 is a schematic flowchart of a third embodiment of a vaporization state recognition method according to this application.
FIG. 7 is a schematic structural diagram of an embodiment of a vaporization state recognition apparatus according to this application.
FIG. 8 is a schematic structural diagram of an embodiment of an electronic device according to this application.
FIG. 9 is a schematic block diagram of an electronic vaporization device according to an embodiment of this application.
FIG. 10 is a schematic structural diagram of a computer-readable storage medium according to an embodiment of this application.

### DETAILED DESCRIPTION

The technical solutions in embodiments of this application are clearly and completely described below with reference to the accompanying drawings in the embodiments of this application. Apparently, the described embodiments are merely some rather than all of the embodiments of this application. All other embodiments obtained by a person of ordinary skill in the art based on the embodiments of this application without creative efforts shall fall within the scope of this application.

In the following description, for the purpose of illustration rather than limitation, specific details such as the specific system structure, interface, and technology are proposed to thoroughly understand this application.

The terms "first", "second", and "third" in this application are merely intended for a purpose of description, and shall not be understood as indicating or implying relative significance or implicitly indicating the number of indicated technical features. Therefore, features defining "first", "second", and "third" may explicitly or implicitly include at least one of the features. In the description of this application, "a plurality of" means at least two, such as two and three unless it is specifically defined otherwise. All directional indications (for example, upper, lower, left, right, front, and back) in the embodiments of this application are only used for explaining relative position relationships, movement situations, or the like between the various components in a specific posture (as shown in the accompanying drawings). If the specific posture changes, the directional indications change correspondingly. In the embodiments of this application, the terms "include", "have", and any variant thereof are intended to cover a non-exclusive inclusion. For example, a process, method, system, product, or device that includes a series of steps or units is not limited to the listed steps or units, but further optionally includes a step or unit that is not listed, or further optionally includes another step or component that is intrinsic to the process, method, product, or device.

"Embodiment" mentioned in this specification means that particular features, structures, or characteristics described with reference to the embodiment may be included in at least one embodiment of this application. The term appearing at different positions of this specification may not refer to the same embodiment or an independent or alternative embodiment that is mutually exclusive with another embodiment. A person skilled in the art explicitly or implicitly understands that the embodiments described in this specification may be combined with other embodiments.

This application is described in detail below with reference to the accompanying drawings and embodiments.

An electronic vaporization device is generally arranged with a sensor such as a probe in a liquid storage cavity. Through the sensor such as the probe being in contact with a to-be-vaporized substrate, the liquid level of the to-be-vaporized substrate may be detected to determine whether there still exists the to-be-vaporized substrate in the liquid storage cavity, so as to determine whether a vaporizer is in a dry heating state. It can be understood that the inventor believes that possible reasons of the vaporizer of the electronic vaporization device being in a dry heating state include: 1. the to-be-vaporized substrate stored in the vaporizer is used up; 2. the to-be-vaporized substrate is poorly liquid-supplied; 3. a driving mode of an atomizing sheet does not match with the state of the existing to-be-vaporized substrate. When the atomizing sheet is in the dry heating state, the atomizing sheet may be damaged, and the temperature of the atomizing sheet may increase, causing the to-be-vaporized substrate (such as medicinal liquid) to be ineffective. However, currently, the dry heating caused by the poor liquid supply of the to-be-vaporized substrate is not detectable in existing detection manners. Based on the existing problems of the related art, this application provides a vaporization state recognition method to more accurately detect whether the vaporization state of the vaporizer is the dry heating.

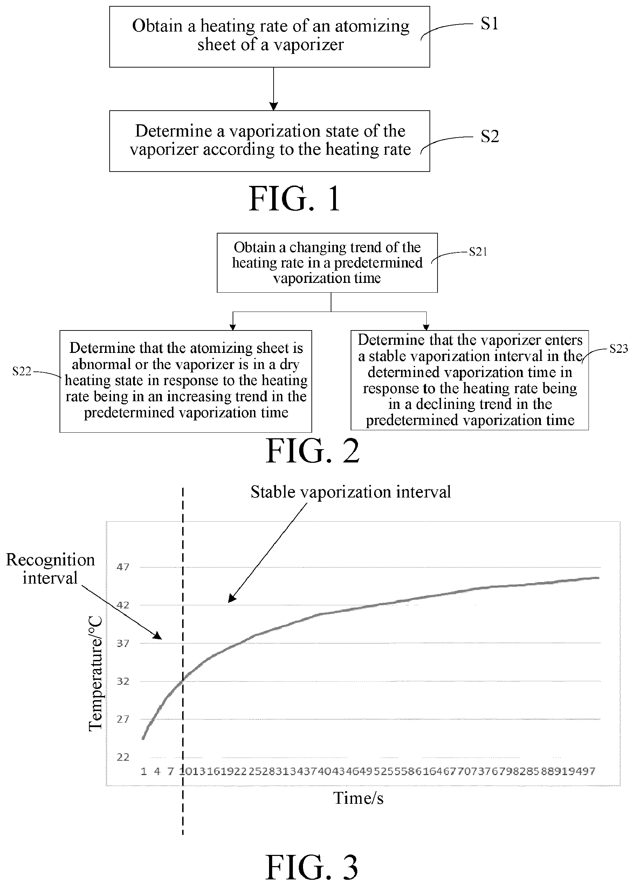

Referring to FIG. 1, FIG. 1 is a schematic flowchart of a first embodiment of a vaporization state recognition method according to this application.

The vaporization state recognition method according to this application specifically includes operations at blocks illustrated herein.

At block S1: Obtain a heating rate of an atomizing sheet of a vaporizer.

Specifically, Step 1 includes: obtaining a temperature of the atomizing sheet; and obtaining the heating rate of the atomizing sheet according to the temperature of the atomizing sheet and a vaporization time corresponding to the atomizing sheet. The temperature of the atomizing sheet may be detected through a temperature characteristic component on the atomizing sheet. The temperature characteristic component may be a positive temperature coefficient thermistor, a negative temperature coefficient thermistor or the like, as long as the temperature of the atomizing sheet can be detected. It can be understood that the atomizing sheet generally includes a piezoelectric ceramic and a metal substrate. The metal substrate may have TCR (temperature coefficient of resistance) performance, so as to obtain the temperature of the metal substrate to obtain the temperature of the atomizing sheet. The obtained temperature of the atomizing sheet is a real-time temperature thereof.

At block S2: Determine a vaporization state of the vaporizer according to the heating rate.

Specifically, the principle of the determining a vaporization state of the vaporizer according to the heating rate is as follows. The inventor of this application finds that when the atomizing sheet is in the dry heating state, the temperature of the atomizing sheet rises rapidly and the temperature rise rate is much greater than the temperature rise rate in normal vaporization; and in a different temperature section, the temperature rise rate is different when the atomizing sheet is in a dry heating state. Therefore, whether the vaporization state of the vaporizer is dry heating is determined through the heating rate of the vaporizer.

Referring to FIG. 2, FIG. 2 is a schematic flowchart of step S2 in the vaporization state recognition method according to FIG. 1. The determining a vaporization state of the vaporizer according to the heating rate specifically includes operations at blocks illustrated herein.

S21: Obtain a changing trend of the heating rate in a predetermined vaporization time.

An electronic vaporization device including an ampoule microporous atomizing sheet is taken as an example for experiments. A temperature rise curve of the atomizing sheet during a normal vaporization is obtained through performing a normal vaporization test on the electronic vaporization device. The experiment result is shown in FIG. 3. FIG. 3 is a temperature rise curve diagram of the atomizing sheet in normal vaporization according to this application. In FIG. 3, an X-axis is the number of sampling points, and a Y-axis is the sampling temperature corresponding to each sampling point; in this experiment, a sampling time interval is 1s, and a total of 97 sampling points are collected. In FIG. 3, the temperature rise curve of the atomizing sheet in normal vaporization is divided into a recognition interval and a stable vaporization interval.

It can be seen from FIG. 3 that a changing rule of the heating rate in the recognition interval is that: when the electronic vaporization device works, the to-be-vaporized substrate is started to be heated and vaporized. With the progress of the vaporization, a temperature difference appears in the to-be-vaporized substrate. The temperature of a part of the to-be-vaporized substrate which is close to the atomizing sheet, is high. While the temperature of a part of the to-be-vaporized substrate which is away from the atomizing sheet, is low. The to-be-vaporized substrate in a low temperature zone cools down the to-be-vaporized substrate in a high temperature zone, which restricts the heating rate of the to-be-vaporized substrate in the high temperature zone. In this case, the heating rate of the atomizing sheet is relatively high. With the further progress of the vaporization, the temperature of the to-be-vaporized substrate is basically the uniform and is close to the temperature of the atomizing sheet. The heating rate of the atomizing sheet gradually tends to be gentle, and the vaporization enters the stable vaporization interval. The heating rate of the atomizing sheet gradually tends to be gentle, representing that the changing trend of the heating rate tends to be gentle, that is, the growth of the heating rate is in a declining trend.

It can be seen from FIG. 3 that after the electronic vaporization device starts the vaporization, after around 10s of the vaporization, the heating rate of the atomizing sheet tends to be gentle, and the electronic vaporization device enters the stable vaporization interval. That is, when the electronic vaporization device is in a normal vaporization state, the electronic vaporization device enters the stable vaporization interval in the predetermined vaporization time (for example, 10s described above) of vaporization, and the heating rate of the atomizing sheet gradually tends to be gentle. The predetermined vaporization time is the time corresponding to the recognition interval. Therefore, whether the vaporization state is dry heating is determined through obtaining the changing trend of the heating rate in the predetermined vaporization time. Since a vaporization parameter is set different in different electronic vaporization devices, the predetermined vaporization time needed for entering the stable vaporization interval is different. The predetermined vaporization time may be set to be a time period of vaporization for 4s to 12s counted from the start of the vaporizer. The start of the vaporizer here means the time when the vaporizer starts to work.

At block S22: Determine that the atomizing sheet is abnormal or the vaporizer is in a dry heating state in response to the heating rate being in an increasing trend in the predetermined vaporization time.

After the electronic vaporization device vaporizes for the predetermined vaporization time, the heating rate of the atomizing sheet is still in an increasing trend, representing that the vaporization does not or will not enter the stable vaporization interval. Therefore, the atomizing sheet is abnormal or in a dry heating state. It can be understood that when the atomizing sheet is abnormal, the to-be-vaporized substrate cannot be heated in a predetermined heating manner, and the vaporization cannot enter the stable vaporization interval after vaporizing for the predetermined vaporization time. When there is no to-be-vaporized substrate or there is little to-be-vaporized substrate in a liquid storage cavity when the electronic vaporization device is started, the dry heating appears before entering the stable vaporization interval.

At block S23: Determine that the vaporizer enters a stable vaporization interval in the determined vaporization time in response to the heating rate being in a declining trend in the predetermined vaporization time.

After the predetermined vaporization time, the heating rate of the atomizing sheet is in a declining trend, representing that the heating rate of the atomizing sheet tends to be gentle, and the vaporization enters the stable vaporization interval. It is the recognition interval before entering the stable vaporization interval, that is to recognize whether the atomizing sheet is abnormal or the atomizing sheet is in the dry heating state when the electronic vaporization device is started.

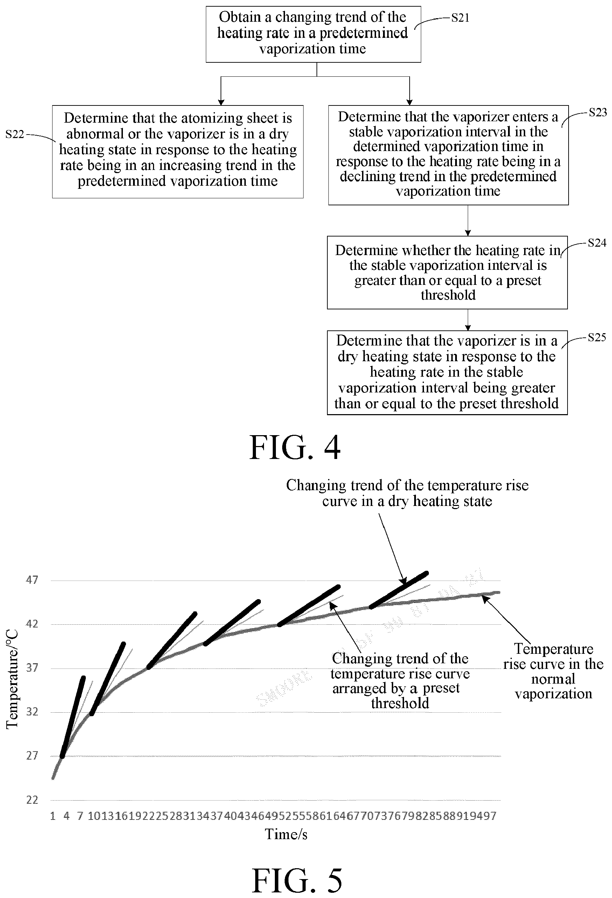

It can be understood that after the vaporization enters the stable vaporization interval, with the progress of the vaporization, the to-be-vaporized substrate is used up and the dry heating may appear. Therefore, after entering the stable vaporization interval, the heating rate of the atomizing sheet is required to be detected continuously to determine whether the vaporization state is dry heating. Based on this, this application provides another embodiment of the vaporization state recognition method. Referring to FIG. 4, FIG. 4 is a schematic flowchart of step S2 of a second embodiment of a vaporization state recognition method according to this application.

In the second embodiment of the vaporization state recognition method, step S1 and step S2 are the same as the step S1 and the step S2 in the first embodiment of the vaporization state recognition method, and details are not described herein again. The difference is that, after step S23, the embodiment further includes operations at blocks illustrated herein.

At block S24: Determine whether the heating rate in the stable vaporization interval is greater than or equal to a preset threshold.

When the atomizing sheet is in the dry heating state, the temperature of the atomizing sheet rises rapidly, that is, whether the vaporization state of the atomizing sheet is dry heating may be determined through arranging the preset threshold and comparing the heating rate of the atomizing sheet with the preset threshold. It can be understood that the real-time heating rate of the atomizing sheet may be compared with the preset threshold.

It can be seen from FIG. 3, when the atomizing sheet is in the normal vaporization state, after entering the stable vaporization interval, different heating rates correspond to different temperatures sub-intervals; and in order to accurately detect whether the vaporization state of the atomizing sheet is dry heating, different temperature sub-intervals are set with different preset thresholds for comparison. That is, in an embodiment, whether the vaporization state is dry heating may be detected through determining whether the heating rate in each different sub-interval in the stable vaporization interval is greater than or equal to the preset threshold corresponding to each sub-interval.

Specifically, an electronic vaporization device including an ampoule microporous atomizing sheet is taken as an example for experiments, through performing a normal vaporization test and a dry heating vaporization test on the electronic vaporization device under a constant power. The temperature rise curve of the atomizing sheet in the normal vaporization and the changing trend of the temperature rise curve of the atomizing sheet in the state of dry heating are obtained, an intermediate value between the heating rate of the normal vaporization and the heating rate of the dry heating is taken as a dry heating recognition preset threshold, and the temperature rise curve in the normal vaporization and the changing trend of the temperature rise curve in the state of dry heating are drawn according to the changing trend of the temperature rise curve arranged by the preset threshold to obtain FIG. 5. FIG. 5 is a temperature rise curve comparison diagram of an atomizing sheet in different vaporization states according to this application. In FIG. 5, an X-axis is the number of sampling points, and a Y-axis is the sampling temperature corresponding to the sampling point; in this experiment, a sampling time interval is 1s, and a total of 97 sampling are is collected. The test is carried out on the atomizing sheet under a condition of a vaporization temperature range of 20°C-60°C.

Exemplarily, the stable vaporization interval is divided into four different sub-intervals, such as 30°C-35°C, 35°C-40°C, 40°C-50°C, and 50°C-60°C. Firstly, the temperature rise curve of the atomizing sheet during the vaporization is obtained through the normal vaporization test, and the heating rates of the above four different sub-intervals are obtained by calculation; further, the heating rates of the atomizing sheet in the above four different sub-intervals during the state of dry heating are obtained; then, for one of the sub-intervals, the intermediate value between the heating rate in the normal vaporization and the heating rate in the state of dry heating is taken as the preset threshold corresponding to the sub-interval. The temperature of the atomizing sheet is 30°C-35°C, and the corresponding preset threshold of the heating rate is 2°C/S. The temperature of the atomizing sheet is 35°C-40°C, and the corresponding preset threshold of the heating rate is 1.5°C/S. The temperature of the atomizing sheet is 40°C-50°C, and the corresponding preset threshold of the heating rate is 1.2°C/S. The temperature of the atomizing sheet is 50°C-60°C, and the corresponding preset threshold of the heating rate is 1°C/S.

It can be understood that in the above test, after the vaporization for 4s to 12s counted from the start of the electronic vaporization device (that is, the time when the electronic vaporization device starts to work), the temperature of the atomizing sheet reaches 30°C, entering the stable vaporization interval at the same time. The division of the different sub-intervals in the stable vaporization interval may be designed according to needs, as long as the preset threshold is greater than the heating rate in the normal vaporization and less than the heating rate in the state of dry heating.

At block S25: Determine that the vaporizer is in a dry heating state in response to the heating rate in the stable vaporization interval being greater than or equal to the preset threshold.

In the stable vaporization interval, the heating rate of the atomizing sheet is greater than or equal to the preset threshold, while the preset threshold is greater than the heating rate in the normal vaporization state, representing that the temperature of the atomizing sheet rises rapidly and the vaporizer is in the dry heating state.

When detecting whether the vaporization state is dry heating through determining whether the heating rate in each different sub-interval in the stable vaporization interval is greater than or equal to the preset threshold corresponding to each sub-interval, it is determined that the vaporizer is in the dry heating state when the real-time heating rate in each different sub-interval is greater than the preset threshold corresponding to each sub-interval.

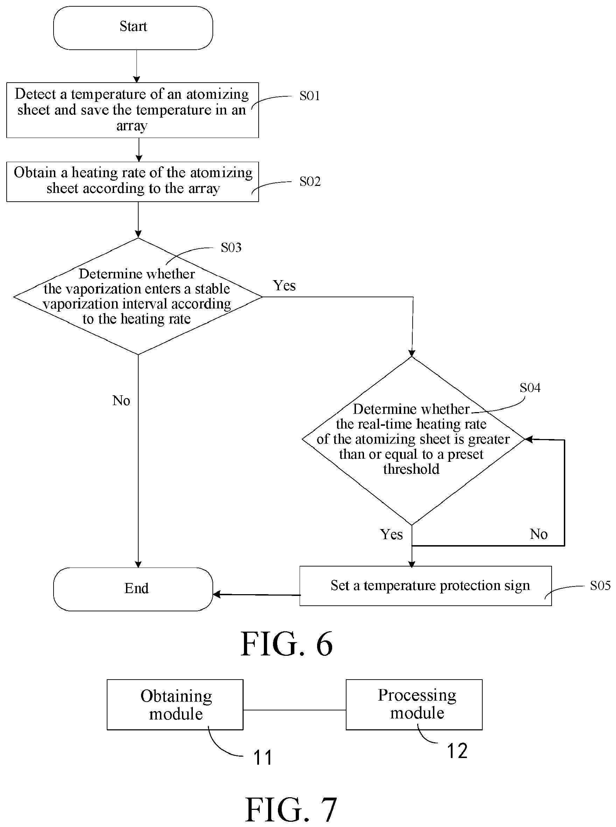

Referring to FIG. 6, FIG. 6 is a schematic flowchart of a third embodiment of a vaporization state recognition method according to this application.

In the third embodiment of the vaporization state recognition method, the method includes operations at blocks illustrated herein.

At block S01: Detect the temperature of the atomizing sheet and save the temperature in an array.

The temperature of the atomizing sheet may be detected through a temperature characteristic component arranged on the atomizing sheet. The temperature characteristic component may be a positive temperature coefficient thermistor, a negative temperature coefficient thermistor or the like, as long as the temperature of the atomizing sheet can be detected. The temperature of the atomizing sheet may be obtained through the TCR performance of the atomizing sheet.

At block S02: Obtain a heating rate of the atomizing sheet according to the array.

The heating rate of the atomizing sheet is obtained through calculating the temperature of the atomizing sheet in the array and the corresponding vaporization time.

At block S03: Determine whether the vaporization enters a stable vaporization interval according to the heating rate.

When the electronic vaporization device starts for a predetermined vaporization time, and the heating rate is in a declining trend, it is determined that the vaporization enters the stable vaporization interval and step S04 is performed; when the electronic vaporization device starts for the predetermined vaporization time, and the heating rate is in an increasing trend, it is determined that the vaporization state of the vaporizer is dry heating or the atomizing sheet is abnormal.

At block S04: Determine whether the real-time heating rate of the atomizing sheet is greater than or equal to a preset threshold.

When the real-time heating rate of the atomizing sheet is greater than or equal to the preset threshold, step S05 is performed; when the real-time heating rate of the atomizing sheet is less than the preset threshold, the determining whether the real-time heating rate is greater than the preset threshold is continuously performed.

In the stable vaporization interval, the heating rate of the atomizing sheet is greater than or equal to the preset threshold, while the preset threshold is greater than the heating rate in the normal vaporization state, representing that the temperature of the atomizing sheet rises rapidly and the vaporizer is in the dry heating state.

When detecting whether the vaporization state is dry heating through determining whether the heating rate in each different sub-interval in the stable vaporization interval is greater than or equal to the preset threshold corresponding to each sub-interval, it is determined that the vaporizer is in the dry heating state when the real-time heating rate in each different sub-interval is greater than the preset threshold corresponding to each sub-interval.

At block S05: Set a temperature protection sign.

The temperature protection sign is set when the atomizing sheet is determined to be in the dry heating state, for example, stopping the vaporizer, so as to prevent a decrease of the performance of the atomizing sheet affecting the performance of the electronic vaporization device caused by a long-term dry heating damaging the atomizing sheet.

Referring to FIG. 7, FIG. 7 is a schematic structural diagram of an embodiment of a vaporization state recognition apparatus according to this application.

The vaporization state recognition apparatus includes an obtaining module 11 and a processing module 12; the obtaining module 11 is configured to obtain a heating rate of an atomizing sheet of a vaporizer, and the processing module 12 is configured to determine a vaporization state of the vaporizer according to the heating rate. The vaporization state recognition apparatus may be configured to implement the vaporization state recognition method according to any one of the above embodiments, and implement an accurate detection on the vaporization state of the vaporizer.

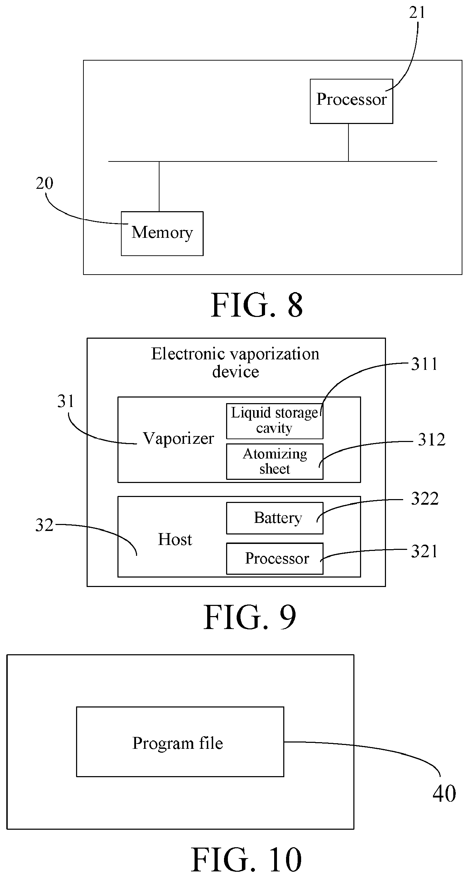

Referring to FIG. 8, FIG. 8 is a schematic structural diagram of an embodiment of an electronic device according to this application. The electronic device includes a memory 20 and a processor 21 connected to each other.

The memory 20 is configured to store and implement program instructions of the vaporization state recognition method according to any one of the above embodiments.

The processor 21 is configured to execute the program instructions stored in the memory 20; that is, the processor 21 reads the program instructions stored in the memory 20 from the memory 20 to execute the vaporization state recognition method according to any one of the above.

The processor 21 may be referred to as a CPU (Central Processing Unit). The processor 21 may be an integrated circuit chip with a signal processing capability. The processor 21 may further be a general-purpose processor, a digital signal processor (DSP), an application specific integrated circuit (ASIC), a field programmable gate array (FPGA) or other programmable logic devices, a discrete gate or a transistor logic device, a discrete hardware component. The general-purpose processor may be a microprocessor, or may be any conventional processor, or the like.

The memory 20 may be a memory stick, a TF card or the like, and may store all information in the electronic device of the device, including input original data, a computer program, an intermediate running result and a final running result, all stored in the memory. The memory 20 stores and reads the information according to a position determined by a controller. With the memory 20, the electronic device has a memory function, so as to ensure a normal operation. The memory 20 of the electronic device is divided into a main memory (internal memory) and an auxiliary memory (external memory) according to the purpose, and there is also a classification method dividing the memory 20 into an external memory and an internal memory. The external memory usually is a magnetic medium or an optical disk or the like, which can store information for a long time. The internal memory refers to the storage component on a mainboard, configured to store data and programs being executed currently, but merely configured to store the programs and the data temporarily. The data is lost when the power is turned off or there is a power failure.

In the several embodiments provided in this application, it should be understood that the disclosed method and apparatus may be implemented in other manners. For example, the described apparatus embodiment is merely exemplary. For example, the division of modules or units is merely a logical function division and may be other division during actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electric, mechanical, or other forms.

The units described as stand-alone components above may be separated physically or not. The components illustrated as units may be physical units or not, and specifically, may be located in one place, or distributed on a plurality of network elements. Some or all of the units may be selected according to actual requirements to achieve the objectives of the solutions of the implementations.

In addition, functional units in the embodiments of this application may be integrated into one processing unit, or each of the units may exist alone physically, or two or more units may be integrated into one unit. The above integrated unit may be implemented in a form of a hardware, or may be implemented in the form of a software functional unit.

When the integrated unit is implemented in the form of a software functional unit and sold or used as an independent product, the integrated unit may be stored in a computer-readable storage medium. Based on such an understanding, the technical solutions of this application essentially, or the part contributing to the related art, or all or some of the technical solutions may be implemented in the form of a software product. The computer software product is stored in a storage medium and includes several instructions for instructing a computer device (which may be a personal computer, a system server, a network device, or the like) or a processor to perform all or some of the steps of the methods in the implementations of this application.

In an embodiment, the electronic device is an electronic vaporization device. The electronic vaporization device is taken as an example for describing the structure of the electronic device in detail. Referring to FIG. 9, FIG. 9 is a schematic block diagram of an electronic vaporization device according to an embodiment of this application.

The electronic vaporization device includes a vaporizer 31 and a host 32. The vaporizer 31 and the host 32 may be detachably connected, or may be integrally formed. The specific of above may be selected according to needs.

The vaporizer 31 includes a liquid storage cavity 311 and an atomizing sheet 312. The liquid storage cavity 311 is configured to store a to-be-vaporized substrate and the atomizing sheet 312 is configured to vaporize the to-be-vaporized substrate. The host 32 includes a processor 321 and a battery 322. The battery 322 provides power for the atomizing sheet 312, and the processor 321 is configured to control whether the battery 322 provides power for the atomizing sheet 312.

In an embodiment, the vaporizer 31 further includes a temperature characteristic component (not shown in drawings) arranged on the atomizing sheet 312. The temperature characteristic component is connected to the processor 321 and is configured to detect the temperature of the atomizing sheet 312. The temperature characteristic component may be a positive temperature coefficient thermistor, a negative temperature coefficient thermistor or the like, as long as the temperature of the atomizing sheet can be detected.

In another embodiment, the atomizing sheet 312 includes a piezoelectric ceramic (not shown in drawings) and a metal substrate (not shown in drawing). The metal substrate has TCR performance, so as to obtain the temperature of the metal substrate to obtain the temperature of the atomizing sheet 312.

The electronic vaporization device may be used for vaporization of liquid substrates such as e-liquid and medicinal liquid. It can be understood that the to-be-vaporized substrate stored in the liquid storage cavity 311 may be the liquid substrates such as e-liquid and medicinal liquid, which is selected according to needs.

Referring to FIG. 10, FIG. 10 is a schematic structural diagram of a computer-readable storage medium according to an embodiment of this application. The storage medium in this application stores a program file 40 which can implement the vaporization state recognition method as above. The program file 40 may be stored in a storage medium in a form of a computer software product and includes several instructions for instructing a computer device (which may be a personal computer, a server, a network device, or the like) or a processor to perform all or some of the steps of the methods described in the implementations of this application. The foregoing storage electronic includes: any medium that can store program code, such as a USB flash disk, a removable hard disk, a read-only memory (ROM), a random access memory (RAM), a magnetic disk or an optical disc, or a terminal device such as a computer, a server, a mobile phone, or a tablet.

The foregoing descriptions are merely implementations of this application, and the patent scope of this application is not limited thereto. All equivalent structure or process changes made according to the content of this specification and accompanying drawings in this application or by directly or indirectly applying this application in other related technical fields shall fall within the protection scope of this application.

## Claims

1. A vaporization state recognition method, comprising:
obtaining (S1) a heating rate of an atomizing sheet of a vaporizer; and
determining (S2) a vaporization state of the vaporizer according to the heating rate;
**characterized in that**, wherein the determining (S2) a vaporization state of the vaporizer according to the heating rate comprises:
obtaining a temperature rise curve of the atomizing sheet in a predetermined vaporization time,
obtaining (S21) a changing trend of the heating rate in the predetermined vaporization time based on the temperature rise curve;
determining (S22) that the atomizing sheet is abnormal or the vaporizer is in a dry heating state in response to the heating rate being in an increasing trend in the predetermined vaporization time; and
determining (S23) that the vaporizer enters a stable vaporization interval in the determined vaporization time in response to the heating rate being in a declining trend in the predetermined vaporization time;
wherein the predetermined vaporization time is a time period counted from a time when the vaporizer starts to work to a time when the vaporizer enters the stable vaporization interval.

2. The method according to claim 1, wherein after the determining (S23) that the vaporizer enters a stable vaporization interval in the determined vaporization time, the method further comprises:
determining (S24) whether the heating rate in the stable vaporization interval is greater than or equal to a preset threshold; and
determining (S25) that the vaporizer is in the dry heating state in response to the heating rate in the stable vaporization interval being greater than or equal to the preset threshold.

3. The method according to claim 2, wherein the determining (S24) whether the heating rate in the stable vaporization interval is greater than or equal to a preset threshold comprises:
determining whether the heating rate in each different sub-interval in the stable vaporization interval is greater than or equal to the preset threshold corresponding to each sub-interval.

4. The method according to claim 3, wherein the present threshold is obtained by:
obtaining a temperature rise curve of the atomizing sheet in a normal vaporization state and a temperature rise curve of the atomizing sheet in the dry heating state, and taking an intermediate value between the heating rate in the normal vaporization state and the heating rate in the dry heating state corresponding to each sub-interval as the preset threshold corresponding to each sub-interval.

5. The method according to claim 2, after the determining (S25) that the vaporizer is in the dry heating state in response to the heating rate in the stable vaporization interval being greater than or equal to the preset threshold, further comprising:
setting (05) a temperature protection sign, comprising: stopping the vaporizer.

6. The method according to claim 1, wherein the predetermined vaporization time is a time period of vaporization for 4s to 12s counted from the vaporizer being started.

7. A vaporization state recognition apparatus, comprising:
an obtaining module (11), configured to obtain a heating rate of an atomizing sheet of a vaporizer; and
a processing module (12), configured to determine a vaporization state of the vaporizer according to the heating rate;
**characterized in that**, wherein when performing the determining a vaporization state of the vaporizer according to the heating rate, the processing module (12) is further configured to:
obtain a temperature rise curve of the atomizing sheet in a predetermined vaporization time;
obtain a changing trend of the heating rate in the predetermined vaporization time;
determine that the atomizing sheet is abnormal or the vaporizer is in a dry heating state in response to the heating rate being in an increasing trend in the predetermined vaporization time; and
determine that the vaporizer enters a stable vaporization interval in the determined vaporization time in response to the heating rate being in a declining trend in the predetermined vaporization time;
wherein the predetermined vaporization time is a time period counted from a time when the vaporizer starts to work to a time when the vaporizer enters the stable vaporization interval.

8. The vaporization state recognition apparatus according to claim 7, wherein after the determining that the vaporizer enters a stable vaporization interval in the determined vaporization time, the processing module (12) is further configured to:
determine whether the heating rate in the stable vaporization interval is greater than or equal to a preset threshold; and
determine that the vaporizer is in the dry heating state in response to the heating rate in the stable vaporization interval being greater than or equal to the preset threshold.

9. An electronic device, comprising: a memory (20) and a processor (21), wherein the memory (20) stores program instructions, and the processor (21) reads the program instructions from the memory (20) to execute the vaporization state recognition method according to any one of claims 1 to 6.

10. The electronic device according to claim 9, wherein the electronic device is an electronic vaporization device, wherein the electronic vaporization device comprises the atomizing sheet (312) and a temperature characteristic component, wherein the temperature characteristic component is connected to the processor (21) and is configured to detect a temperature of the atomizing sheet (312).

11. The electronic device according to claim 10, wherein the temperature characteristic component is a positive temperature coefficient thermistor or a negative temperature coefficient thermistor.

12. A computer-readable storage medium, storing a program file (40), wherein the program file (40) is configured to be executed by a processor to implement the vaporization state recognition method according to any one of claims 1 to 6.

## Patentansprüche

1. Verfahren zur Erkennung des Verdampfungszustands, umfassend:
das Ermitteln (S1) einer Heizrate eines Zerstäubungsblatts eines Verdampfers; und
das Bestimmen (S2) eines Verdampfungszustands des Verdampfers anhand der Heizrate;
**dadurch gekennzeichnet, dass** das Bestimmen (S2) des Verdampfungszustands des Verdampfers anhand der Heizrate umfasst:
das Ermitteln einer Temperaturanstiegskurve des Zerstäubungsblatts innerhalb einer vorgegebenen Verdampfungszeit,
das Ermitteln (S21) eines Änderungstrends der Heizrate innerhalb der vorgegebenen Verdampfungszeit basierend auf der Temperaturanstiegskurve,
das Bestimmen (S22), dass das Zerstäubungsblatt abnormal ist oder sich der Verdampfer in einem Trockenheizzustand befindet, als Reaktion darauf, dass die Heizrate innerhalb der vorgegebenen Verdampfungszeit einen steigenden Trend aufweist; und
das Bestimmen (S23), dass der Verdampfer innerhalb der vorgegebenen Verdampfungszeit in einen stabilen Verdampfungsbereich eintritt, als Reaktion darauf, dass die Heizrate innerhalb der vorgegebenen Verdampfungszeit einen abnehmenden Trend aufweist;
wobei die vorgegebene Verdampfungszeit ein Zeitabschnitt ist, der von dem Zeitpunkt, zu dem der Verdampfer zu arbeiten beginnt, bis zu dem Zeitpunkt, zu dem der Verdampfer in den stabilen Verdampfungsbereich eintritt, gezählt wird.

2. Verfahren gemäß Anspruch 1, wobei nach dem Bestimmen (S23), dass der Verdampfer in den stabilen Verdampfungsbereich innerhalb der vorgegebenen Verdampfungszeit eintritt, das Verfahren ferner umfasst:
das Bestimmen (S24), ob die Heizrate im stabilen Verdampfungsbereich größer als oder gleich einem voreingestellten Schwellenwert ist; und
das Bestimmen (S25), dass sich der Verdampfer im Trockenheizzustand befindet, als Reaktion darauf, dass die Heizrate im stabilen Verdampfungsbereich größer als oder gleich dem voreingestellten Schwellenwert ist.

3. Verfahren gemäß Anspruch 2, wobei das Bestimmen (S24), ob die Heizrate im stabilen Verdampfungsbereich größer als oder gleich einem voreingestellten Schwellenwert ist, folgendes umfasst:
das Bestimmen, ob die Heizrate in jedem der verschiedenen Unterabschnitte, die im stabilen Verdampfungsbereich enthalten sind, größer als oder gleich dem jeweils für den Unterabschnitt voreingestellten Schwellenwert ist.

4. Verfahren gemäß Anspruch 3, wobei der voreingestellte Schwellenwert erhalten wird durch:
das Ermitteln einer Temperaturanstiegskurve des Zerstäubungsblatts im normalen Verdampfungszustand und einer Temperaturanstiegskurve des Zerstäubungsblatts im Trockenheizzustand, und
das Nehmen eines Zwischenwerts zwischen der Heizrate im normalen Verdampfungszustand und der Heizrate im Trockenheizzustand, der jeweils dem jeweiligen Unterabschnitt entspricht, als den für jeden Unterabschnitt voreingestellten Schwellenwert.

5. Verfahren gemäß Anspruch 2, wobei nach dem Bestimmen (S25), dass sich der Verdampfer im Trockenheizzustand befindet, als Reaktion darauf, dass die Heizrate im stabilen Verdampfungsbereich größer als oder gleich dem voreingestellten Schwellenwert ist, das Verfahren ferner umfasst:
das Setzen (S5) eines Temperaturschutzsignals, welches das Abschalten des Verdampfers umfasst.

6. Verfahren gemäß Anspruch 1, wobei die vorgegebene Verdampfungszeit ein Verdampfungszeitraum von 4 Sekunden bis 12 Sekunden ist, gerechnet ab dem Startzeitpunkt des Verdampfers.

7. Vorrichtung zur Erkennung des Verdampfungszustands, umfassend:
ein Erfassungsmodul (11), konfiguriert zum Ermitteln einer Heizrate eines Zerstäubungsblatts eines Verdampfers; und
ein Verarbeitungsmodul (12), konfiguriert zum Bestimmen eines Verdampfungszustands des Verdampfers anhand der Heizrate;
**dadurch gekennzeichnet, dass** beim Ausführen des Bestimmens des Verdampfungszustands des Verdampfers anhand der Heizrate das Verarbeitungsmodul (12) ferner konfiguriert ist,
eine Temperaturanstiegskurve des Zerstäubungsblatts während einer vorgegebenen Verdampfungszeit zu ermitteln;
einen Änderungstrend der Heizrate während der vorgegebenen Verdampfungszeit zu ermitteln;
zu bestimmen, dass das Zerstäubungsblatt abnormal ist oder sich der Verdampfer in einem Trockenheizzustand befindet, als Reaktion darauf, dass die Heizrate während der vorgegebenen Verdampfungszeit einen steigenden Trend aufweist; und
zu bestimmen, dass der Verdampfer während der vorgegebenen Verdampfungszeit in einen stabilen Verdampfungsbereich eintritt, als Reaktion darauf, dass die Heizrate während der vorgegebenen Verdampfungszeit einen abnehmenden Trend aufweist;
wobei die vorgegebene Verdampfungszeit ein Zeitraum ist, der vom Zeitpunkt, zu dem der Verdampfer zu arbeiten beginnt, bis zu dem Zeitpunkt, zu dem der Verdampfer in den stabilen Verdampfungsbereich eintritt, gezählt wird.

8. Vorrichtung zur Erkennung des Verdampfungszustands gemäß Anspruch 7, wobei nach der Bestimmung, dass der Verdampfer während der vorgegebenen Verdampfungszeit in einen stabilen Verdampfungsbereich eintritt, das Verarbeitungsmodul (12) ferner konfiguriert ist,
zu bestimmen, ob die Heizrate im stabilen Verdampfungsbereich größer oder gleich einem voreingestellten Schwellenwert ist; und
zu bestimmen, dass sich der Verdampfer im Trockenheizzustand befindet, als Reaktion darauf, dass die Heizrate im stabilen Verdampfungsbereich größer oder gleich dem voreingestellten Schwellenwert ist.

9. Elektronisches Gerät, umfassend:
einen Speicher (20); und einen Prozessor (21),
wobei der Speicher (20) Programmanweisungen speichert, und der Prozessor (21) die Programmanweisungen aus dem Speicher (20) liest, um das Verfahren zur Erkennung des Verdampfungszustands gemäß einem der Ansprüche 1 bis 6 auszuführen.

10. Elektronisches Gerät gemäß Anspruch 9, wobei das elektronische Gerät ein elektronisches Verdampfungsgerät ist, das das Zerstäubungsblatt (312) und eine temperaturcharakteristische Komponente umfasst, wobei die temperaturcharakteristische Komponente mit dem Prozessor (21) verbunden ist und konfiguriert ist, die Temperatur des Zerstäubungsblatts (312) zu erfassen.

11. Elektronisches Gerät gemäß Anspruch 10, wobei die temperaturcharakteristische Komponente ein Thermistor mit positivem Temperaturkoeffizienten oder ein Thermistor mit negativem Temperaturkoeffizienten ist.

12. Computerlesbares Speichermedium, das eine Programmdatei (40) speichert, wobei die Programmdatei (40) so konfiguriert ist, dass sie von einem Prozessor ausgeführt wird, um das Verfahren zur Erkennung des Verdampfungszustands gemäß einem der Ansprüche 1 bis 6 umzusetzen.

## Revendications

1. Procédé de reconnaissance d'état de vaporisation, **caractérisé en ce qu'**il comprend :
l'obtention (S1) d'un taux de chauffage d'une feuille d'atomisation d'un dispositif de vaporisation ; et
la détermination (S2) d'un état de vaporisation du dispositif de vaporisation en fonction du taux de chauffage ;
**caractérisé en ce que** ladite détermination (S2) d'un état de vaporisation du dispositif de vaporisation en fonction du taux de chauffage comprend :
l'obtention d'une courbe d'élévation de température de ladite feuille d'atomisation pendant un temps de vaporisation prédéterminé,
l'obtention (S21) d'une tendance d'évolution du taux de chauffage pendant ledit temps de vaporisation prédéterminé, sur la base de ladite courbe d'élévation de température,
la détermination (S22) que ladite feuille d'atomisation est anormale ou que le dispositif de vaporisation est dans un état de chauffage à sec en réponse au fait que le taux de chauffage présente une tendance à l'augmentation pendant ledit temps de vaporisation prédéterminé, et
la détermination (S23) que le dispositif de vaporisation entre dans un intervalle de vaporisation stable pendant ledit temps de vaporisation prédéterminé en réponse au fait que le taux de chauffage présente une tendance à la baisse pendant ledit temps de vaporisation prédéterminé ;
dans lequel le temps de vaporisation prédéterminé est une période de temps comptée à partir du moment où le dispositif de vaporisation commence à fonctionner jusqu'au moment où le dispositif de vaporisation entre dans l'intervalle de vaporisation stable.

2. Le procédé selon la revendication 1, dans lequel, après la détermination (S23) que le dispositif de vaporisation entre dans un intervalle de vaporisation stable pendant le temps de vaporisation prédéterminé, le procédé comprend en outre :
la détermination (S24) de savoir si le taux de chauffage dans l'intervalle de vaporisation stable est supérieur ou égal à un seuil prédéfini ; et
la détermination (S25) que le dispositif de vaporisation est dans l'état de chauffage à sec en réponse au fait que le taux de chauffage dans l'intervalle de vaporisation stable est supérieur ou égal au seuil prédéfini.

3. Le procédé selon la revendication 2, dans lequel la détermination (S24) de savoir si le taux de chauffage dans l'intervalle de vaporisation stable est supérieur ou égal à un seuil prédéfini comprend :
la détermination de savoir si le taux de chauffage dans chacun des différents sous-intervalles compris dans l'intervalle de vaporisation stable est supérieur ou égal au seuil prédéfini correspondant à chaque sous-intervalle.

4. Le procédé selon la revendication 3, dans lequel le seuil prédéfini est obtenu par :
l'obtention d'une courbe d'élévation de température de la feuille d'atomisation dans un état de vaporisation normal et d'une courbe d'élévation de température de la feuille d'atomisation dans l'état de chauffage à sec, et
la prise d'une valeur intermédiaire entre le taux de chauffage dans l'état de vaporisation normal et le taux de chauffage dans l'état de chauffage à sec correspondant à chacun des sous-intervalles comme seuil prédéfini correspondant à chacun des sous-intervalles.

5. Le procédé selon la revendication 2, dans lequel, après la détermination (S25) que le dispositif de vaporisation est dans l'état de chauffage à sec en réponse au fait que le taux de chauffage dans l'intervalle de vaporisation stable est supérieur ou égal au seuil prédéfini, le procédé comprend en outre :
la mise en place (S5) d'un signal de protection de température, comprenant : l'arrêt du dispositif de vaporisation.

6. Le procédé selon la revendication 1, dans lequel le temps de vaporisation prédéterminé est une période de vaporisation comprise entre 4 secondes et 12 secondes, comptée à partir du démarrage du dispositif de vaporisation.

7. Dispositif de reconnaissance d'état de vaporisation, comprenant :
un module d'obtention (11), configuré pour obtenir un taux de chauffage d'une feuille d'atomisation d'un dispositif de vaporisation ; et
un module de traitement (12), configuré pour déterminer un état de vaporisation du dispositif de vaporisation en fonction du taux de chauffage ;
**caractérisé en ce que**, lors de la détermination d'un état de vaporisation du dispositif de vaporisation en fonction du taux de chauffage, le module de traitement (12) est en outre configuré pour :
obtenir une courbe d'élévation de température de la feuille d'atomisation pendant un temps de vaporisation prédéterminé ;
obtenir une tendance d'évolution du taux de chauffage pendant ledit temps de vaporisation prédéterminé ;
déterminer que la feuille d'atomisation est anormale ou que le dispositif de vaporisation est dans un état de chauffage à sec en réponse au fait que le taux de chauffage présente une tendance à l'augmentation pendant ledit temps de vaporisation prédéterminé ; et
déterminer que le dispositif de vaporisation entre dans un intervalle de vaporisation stable pendant ledit temps de vaporisation prédéterminé en réponse au fait que le taux de chauffage présente une tendance à la baisse pendant ledit temps de vaporisation prédéterminé ;
dans lequel le temps de vaporisation prédéterminé est une période de temps comptée à partir du moment où le dispositif de vaporisation commence à fonctionner jusqu'au moment où le dispositif de vaporisation entre dans l'intervalle de vaporisation stable.

8. Le dispositif de reconnaissance d'état de vaporisation selon la revendication 7, dans lequel, après la détermination que le dispositif de vaporisation entre dans un intervalle de vaporisation stable pendant le temps de vaporisation prédéterminé, le module de traitement (12) est en outre configuré pour :
déterminer si le taux de chauffage dans l'intervalle de vaporisation stable est supérieur ou égal à un seuil prédéfini ; et
déterminer que le dispositif de vaporisation est dans l'état de chauffage à sec en réponse au fait que le taux de chauffage dans l'intervalle de vaporisation stable est supérieur ou égal au seuil prédéfini.

9. Dispositif électronique, comprenant :
une mémoire (20) ; et
un processeur (21),
dans lequel la mémoire (20) stocke des instructions de programme, et le processeur (21) lit les instructions de programme depuis la mémoire (20) pour exécuter le procédé de reconnaissance d'état de vaporisation selon l'une quelconque des revendications 1 à 6.

10. Dispositif électronique selon la revendication 9, dans lequel le dispositif électronique est un dispositif de vaporisation électronique, comprenant la feuille d'atomisation (312) et un composant caractéristique de température, ledit composant caractéristique de température étant relié au processeur (21) et configuré pour détecter une température de la feuille d'atomisation (312).

11. Le dispositif électronique selon la revendication 10, dans lequel le composant caractéristique de température est une thermistance à coefficient de température positif ou une thermistance à coefficient de température négatif.

12. Support de stockage lisible par ordinateur, stockant un fichier de programme (40), dans lequel le fichier de programme (40) est configuré pour être exécuté par un processeur afin de mettre en œuvre le procédé de reconnaissance d'état de vaporisation selon l'une quelconque des revendications 1 à 6.
